# EUROPEAN PATENT APPLICATION

(11) **EP 2 286 802 A1**
(43) Date of publication of application: **23.02.2011**
(21) Application number: 10179084.8
(22) Date of filing: 10.10.2006
(51) Int. Cl.: A61K 9/70, A61K 31/27

(54) **Transdermal therapeutic system**

(30) Priority: 01.12.2005 US 741511 P
(62) Divisional of application: 06816633.9
(71) Applicant: Novartis AG, 4056 Basel (CH); Novartis Pharma GmbH, 1230 Wien (AT); LTS LOHMANN Therapie-Systeme AG, 56626 Andernach (DE)
(72) Inventor: Gargiulo, Paul M., New York, NY 10128 (US); Lane, Roger Michael, New York, NY 10025 (US); Platt, Beatrix, 56745, Hausten (DE); Theobald, Frank, 53498, Bad Breisig (DE); Wall, Bettina, 56567, Neuwied-Niederbieber (DE)
(74) Representative: Hutchison, John Robert

(57) **Abstract**

The present invention relates to Transdermal Therapeutic Systems having a silicone adhesive layer, to Transdermal Therapeutic Systems providing specific plasma concentrations, to their manufacture and use.

## Description

The present invention relates to Transdermal Therapeutic Systems comprising a backing layer, a reservoir layer and an adhesive layer, to Transdermal Therapeutic Systems having specific release profiles, to their manufacture and use.

Transdermal Therapeutic Systems (TTS) and their manufacture are generally known in the art. EP 1047409 discloses a TTS containing rivastigmine and an antioxidant. GB 2203040 discloses a TTS containing rivastigmine and a hydrophilic polymer.

These TTS have valuable properties. However, there is a need for further TTS showing improved properties. In particular, there is a need to provide TTS to improve compliance, adhesion, tolerability and / or safety.

Thus, it is an aim of the present invention to provide TTS with improved compliance, adhesion, tolerability a and / or safety properties.

It is a further objective of the present invention to provide a TTS that has a relatively large amount of active ingredient and has an adhesive force to ensure safe application over the entire application period.

It is a further objective of the present invention to provide a TTS that has a relatively large amount of active ingredient without having an inadequately large expanse.

It is a further objective of the present invention to provide a TTS that shows improved adhesive properties without changing the release profile of the active ingredient.

It is a further objective of the present invention to provide a method of treatment and controlled-release formulation(s) that substantially improves the efficacy and tolerability of rivastigmine.

It is a further objective of the present invention to provide a method of treatment and controlled-release formulation(s) that substantially reduces the time and resources needed to administer rivastigmine for therapeutic benefit.

It is a further objective of the present invention to provide a method of treatment and controlled-release formulation(s) that substantially improves compliance with rivastigmine therapy.

It is a further objective of the present invention to provide a method of treatment and controlled-release formulation(s) that have substantially less inter-individual variation with regard to plasma concentrations of rivastigmine required to produce a therapeutic benefit without unacceptable side effects.

This is achieved by a TTS as defined in claim 1 and depending claims.
Figure 1 shows a bar chart illustrating the different adhesive forces of a TTS having an additional silicone adhesive layer (TTS #2) and of a TTS having no additional silicone adhesive layer (TTS #1).
Figure 2 shows a graph illustrating the different permeation rates of rivastigmine through full-thickness human skin, administered by means of a TTS having an additional silicone adhesive layer (TTS #2) or a TTS having no additional silicone adhesive layer (TTS #1).
Figure 3 shows a graph illustrating the different permeation rates of rivastigmine through an EVA membrane, administered by means of a TTS having an additional silicone adhesive layer (TTS #2) or a TTS having no additional silicone adhesive layer (TTS #1).
Figure 4 shows a graph illustrating the plasma PK profiles following capsule (above) or TTS#2 (below) administration

Tests with active ingredients for the treatment of Alzheimer's disease have surprisingly shown that a line of silicone adhesive can be applied to a poorly adhesive reservoir matrix, thus significantly increasing the adhesive properties of the preparation without affecting the thermodynamic properties of the TTS, i.e. without reducing the release of active ingredient from the matrix and its permeation through the skin.

The findings of the tests on transdermal application of active ingredients for the treatment of Alzheimer's disease carried out by the applicant can of course be transferred to other groups of active ingredients. It can therefore be stated in general that for many active ingredients an increasing proportion of active ingredient in the adhesive polymer matrix of the TTS significantly reduces the adhesive properties of the TTS if said active ingredients are solid at room temperature. Usually, if the active ingredients are in a liquid state at room temperature large amounts of so-called "thickening polymers" (e.g. cellulose or polyacrylate derivatives) have to be added in order to achieve mechanical processability of the polymers, which results also in a reduction of adhesive properties

The present invention provides TTS comprising a backing layer, a reservoir layer containing at least one active ingredient and a polymer, an adhesive layer comprising a silicone polymer and a tackifier.

A TTS according to the invention shows improved adhesive properties. Further, and very surprisingly, the so obtained TTS has essentially the same release profile when compared with a standard TTS.

The present invention is further related to a method for substantially improving the efficacy and tolerability of rivastigmine, comprising application of a TTS in the range of 2 to 50 cm², said formulation providing a mean maximum plasma concentration of about 1 to 30 ng/mL from a mean of about 2 to 16 hours after application and an AUC₂₄ₕ of about 25 to 450 ng-h/mL after repeated "QD" (i.e., once daily) administration.

A TTS according to the invention quite surprisingly shows improved tolerability, particularly gastrointestinal adverse events such as nausea and vomiting, relative to equivalent levels of exposure (AUC₂₄ₕ) of Exelon^{®} capsule.

Unless indicated otherwise, the expressions used in this invention have the following meaning:

The term "transdermal therapeutic system" denotes any device that is capable to release a pharmaceutically active ingredient through the skin. This includes particularly self-adhesive devices such as patches.

The term "backing layer" denotes the layer remote from the skin. This layer is preferably active ingredient-impermeable. Any suitable material or combination of materials may be used. For example Polyethylen-therephthalate (PET), Polyethylen, Polylpropylen, Polyurethane, etc. may be employed.

The term "reservoir layer" denotes a layer containing one or more active ingredients in connection with one ore more polymers. In a preferred embodiment, the reservoir layer comprises an active ingredient in the form of a polymer matrix

The term "adhesive layer" denotes the layer facing the skin. This layer comprises a silicon polymer and a tackifier.

The term "detachable protective layer" denotes the layer remote from the patch prior to its application to the skin. This layer is preferably active ingredient-impermeable. Any suitable material or combination of materials may be used. For example siliconized PET, siliconized Polypropylen, siliconized Polyethylen, fluor-polymer coated PET, fluor-polymer coated Polypropylen, Fluor-polymer coated Polyethylen, etc. may be employed.

The term "active ingredient" denotes any active ingredient suitable for transdermal administration. Active ingredients include water-soluble and also water-insoluble, pharmaceutical active ingredients, which may be inorganic or organic substances. Preferred are organic substances. The active ingredients are to be used in accordance with their indication as analgesics, antipyretics, antirheumatics, sedatives, hypnotic agents, antiepileptics, depressants and stimulants, anaesthetics, neuroleptic analgesics, antihistamines, antihypertensive agents, anticoagulants, antithrombotic agents, psychopharmacological agents, psycholeptics, chemotherapeutic agents, e.g. antibiotics, sulphonamides, antituberculosis agents (tuberculostatic agents) or also chemotherapeutic agents against tropical infections, diuretics, spasmolytics, cardiovascular agents, e.g. sympathomimetics, antihypertensive agents, cardiac stimulants, e.g. cardiac glycosides and digitaloids, parenteral sugar therapeutics, analeptics acting on the central nervous system, geriatric agents, tonolytics (of striated muscles), anti-Parkinson agents, cytostatic agents, immunosuppressants, tonics and vitamins, according to B. Helwig (Moderne Arzneimittel), 1980.

Preferably active ingredients are selected from the group consisting of a-adrenoreceptor agonists, β-adrenoreceptor agonists, α-adrenoreceptor blockers, anesthetic analgetics, non-anesthetic analgetics, androgens, anesthetics, antiallergics, antiandrogens, antianginals, antiarrhythmics, penicillins, antidiabetics, antihistaminics, antimigraine agents, hydrated ergot alkaloids, Ca++ antagonists, serotonin antagonists, platelet aggregation inhibitors, antidepressants, broncholytics, estrogens, gestagens, vasodilators, hormones, anti-dementia drugs (including cholinesterase inhibitors).

Preferred antibiotics include penicillin, tetracycline, chlorotetracycline, bacitracin, nystatin, streptomycin, neomycin, polymicin, gramicidin, oxytetracyclin, chloramphenicol, erythromycin, rifampicin, cefazolin, cefoxitin, cefsulodin, cefotiam and mefoxin . Preferred chemotherapeutic agents include sulfamethazine, sulfamerazine, sulfamethizole and sulfisoxazole. Preferred sedatives and hypnotic agents include chloral hydrate, pentabarbital, phenobarnital, secobarbital, codeine and carbroma. Preferred cardiac glycosides and digitaloids include digitoxin and digoxin. Preferred sympathomimetics include epinephrine.

In particular, antipyretics, analgesics and antirheumatics may be used as the active ingredient in the presentation according to the invention in suitable water-soluble form or water-insoluble form, for example propyphenazone, aminophenazone, aspirin (ASA), antipyrine, methyl nifenazine, melaminsulfone, sulfenazone, phenacetin, pentazocine, lactophenin, paracetamol, quinine, flufenamic acid, mefenamic acid, tolfenamic acid, meclofenamic acid, niflumic acid, clonixin or clonixidin, flunixin, ibuprofen, suprofen, ketoprofen, fenoprofen, pirprofen, diclofenac, ibufenac, procticic acid, naproxen, cicloprofen, tolmetin, clopirac, tiaprofenic acid, oxaprozin, fenclozic acid, fentiazac, clidanac, fenclonac, fenoprofen, flurbiprofen, carprofen, sulindac, cinmetacin, fenbuten, etodolac, butifufen.

Preferred psychopharmacological agents include neuroleptics, antidepressants, thymoleptics, thymerethical drugs and tranquilisers such as thioridazine, imipramine, desimipramine, clomipramine, ketimipramine, opipramol, amitriptyline, nortriptyline, reserpine, aromazine, chlorpromazine, fluopromazine, methopromazine, trimeprazine, diethazine, promethazine, aminopromazine, mepazine, pipamazine , maprotiline and memantine.

Preferred antihypertensive agents include oxprenolol and metoprolol.

Preferably, active ingredients are selected from the group of anti-demantia drugs, such as rivastigmine, donepezil, galanthamine, selegiline memanitine and the pharmacologically acceptable salts of said active ingredients.

Preferred cholinesterase inhibitors include tacrine, rivastigmine, donepezil, galantamine, physostigmine, huperzine A and pharmacologically acceptable salts thereof.

Preferred is a combination of rivastigmine and Memantine as active ingredients.

Most preferred active ingredients are chosen from the group consisting of rivastigmine and rivastigmine hydrogentartrate. rivastigmine (Exelon®) is useful in the treatment of patients with mild to moderately severe dementia of the Alzheimer type (also known as Alzheimer's Disease), dementia associated with Parkinson's disease and symptoms of traumatic brain injury.

The term "polymers", when used in connection with the reservoir layer of the active ingredient, denotes a polymer selected from the group consisting of polydimethylsiloxanes, poly-acrylates, , poly-isobutene, polybutenes and styrene-isoprene-styrene block copolymers or mixtures thereof, respectively combined with resins.

Preferred polymers to be used within the reservoir layer are selected from the group consisting of polyacrylates e.g. Durotak 2353 from National Starch.

The term "silicon polymer" denotes polydimethylsiloxane based polymers e.g. the amincompatible Bio-PSA Q7-4302 from Dow Corning.

The term "tackifier" denotes a substance which is increasing the adhesivity/tackiness of the transdermal formulation. Preferred tackifiers are selected from the group consisting of Silicone oils, glycerine esters of hydrogenated resin acids, hydroabietyl alcohol, resin esters, Hydrogenated Methyl Ester of Wood Rosin, Ester of Partially Hydrogenated Wood Rosin Esters of Rosin, etc. and combinations of those. As appreciated by the skilled person, TTS are made out of several layers having specific characteristics. These layers may vary with respect to the individual composition and to the thickness of the separate layers.

In a preferred embodiment of the present invention, the active ingredients used have a low saturation solubility in the silicone adhesive. The saturation solubility of the active ingredient in the silicone adhesive is for example less than 15%-wt., preferably less than 10%-wt., and most preferred between 2 and 8%-wt.

The silicone adhesive layer preferably reduces the active ingredient permeation from the reservoir layer through the skin by no more than 40 %, especially preferably by no more than 20% and more especially preferably by no more than 10%.

The weight per unit area of the silicone adhesive layer is for example in the range of 5 to 60 g/m², preferably in the range of 10 to 30 g/m².

The composition according to the invention may be used for administrating a wide variety of active agents. Suitable active ingredients are the ones identified above.

In a preferred embodiment, the reservoir layer further comprises auxiliaries such as fillers, antioxidants, colorants, skin penetration promoters and / or preservatives. Such auxiliaries are known to the expert and may be selected from standard text books, see in particular Fiedler's "Lexicon der Hilfstoffe", 4th Edition, ECV Aulendorf 1996 and "Handbook of Pharmaceutical Excipients" Wade and Weller Ed.(1994) the contents of which are incorporated herein by reference.

In a particularly preferred embodiment, the reservoir layer contains an antioxidant, such as α-tocopherol, Ascorbyl palmitate or butylated hydroxytoluene (BHT).

In a preferred embodiment, the reservoir layer contains a skin penetration promoter such as Transcutol, Glycerine, Glycerine-esters, Fatty-acids, Salts of Fatty-acids, Azone, Diethyltoluolamide, Propylengylcol, Propylenglycol-esters, Butandiol, Isopropyl-esters, Urea, etc..

In a preferred embodiment, the ratio of thickness of reservoir layer : adhesive layer is in the between of 5:1 and 1: 2; preferably between 2:1 to 1:1.

In a preferred embodiment, the TTS has an adhesive force > 5 N/10 cm² preferably > 10 N/10 cm^{2.}. In a preferred embodiment, the TTS has an adhesive force < 100 N/10 cm² preferably < 50 N/10 cm² The adhesive force is determined according to standard procedures, e.g. as described in the examples.

In a preferred embodiment, the TTS has a size range of 2 to 50 cm², particularly preferred 5 to 20 cm².

In a preferred embodiment, the TTS provides a mean maximum plasma concentration of rivastigmine of 1 to 30 ng/mL from a mean of 2 to 16 hours after application with an AUC₂₄ₕ of 25 to 450 ng-h/mL, particularly preferred, the TTS provides a mean maximum plasma concentration of rivastigmine of 2.5 to 20 ng/mL from a mean of 4 to 12 hours after application with an AUC₂₄ₕ of 45 to 340 ng-h/mL.

In a further embodiment not only the polymer matrix contains the active ingredient(s) but also the silicone adhesive layer.

In a further aspect, the invention provides a TTS which incorporates as active agent a cholinesterase inhibitor in free or pharmaceutically acceptable salt form, for use in the prevention, treatment or delay of progression of dementia.

In a further aspect, the invention provides a method for the prevention, treatment or delay of progression of dementia associated with Parkinson's disease in a subject in need of such treatment, which comprises administering to said subject a therapeutically effective amount of a TTS which incorporates as active agent a cholinesterase inhibitor in free or pharmaceutically acceptable salt form.

In a further aspect, the invention provides a method for the prevention, treatment or delay of progression of Alzheimer's disease in a subject in need of such treatment, which comprises administering to said subject a therapeutically effective amount of a TTS which incorporates as active agent a cholinesterase inhibitor in free or pharmaceutically acceptable salt form.

The manufacturing of a TTS according to the invention may be accomplished in any method known to the skilled person.

In a further aspect, the invention provides a preferred method for manufacturing a TTS. This method comprises the steps of
a.) manufacturing of the active ingredient in adhesive solution
b.) coating of the active ingredient in adhesive solution
c.) drying of the active ingredient in adhesive solution
d.) manufacturing of the silicone adhesive solution
e.) coating of the silicone adhesive solution
f.) laminating of the silicone adhesive layer to the drug in adhesive layer
g.) Punching and Pouching

In a further aspect, the invention provides a TTS comprising as active ingredient rivastigmine in free base or pharmaceutically acceptable salt form and providing specific plasma concentrations.

Little has been published in detail on rivastigmine's biopharmaceutical properties in humans. It is rapidly and completely absorbed. We have found that it is metabolised mainly through hydrolysis by esterases, e.g., acetyl and butyryl cholinesterase and has a plasma half life of 1 hour. It is subject to pre-systemic and systemic metabolism. We now have found that a TTS containing rivastigmine may be produced with advantageous properties, e.g., better tolerability.

The invention thus provides a TTS comprising as active ingredient rivastigmine in free base or pharmaceutically acceptable salt form having a mean maximum plasma concentration of about 1 to 30 ng/ml from a mean of about 2 to 16 hours after application.

The invention further provides a TTS comprising as active ingredient rivastigmine in free base or pharmaceutically acceptable salt form having a mean maximum plasma concentration of about 1 to 30 ng/ml from a mean of about 2 to 16 hours after application and an AUC 24h of about 25 to 450 ng*h/mL after repeated "QD" (i.e. once daily) administration.

A person skilled in art is familiar how to produce a TTS having the above defined plasma profiles. A person skilled in art will appreciate that such plasma profiles may be obtained by varying, e.g.,:
- the composition of the first and/or second components, e.g., the nature and amount of excipients and/or active agent(s)
- the type of the adhesive layer
- the dimension of the patch

A TTS may be formulated with following aspects in mind:
- the time until the release of active agent (lag time or delay time)
- the rate of release of active agent (fast or slow)
- the duration of release of active agent (long or short)
- Reducing first-pass metabolism
- Improve compliance of the patients
- Reduce application intervals

Such aspects may be observed in standard in vitro dissolution tests, e.g., in water or if desired in body fluids, e.g., artificial gastric juices.

Little has been published on reliable time-controlled release formulations allowing a release at a pre-determined time of a single or repeated doses of active agents. There exists a need for such formulations which are commercially acceptable.

After extensive testing, we have now found that it is possible to produce a TTS capable of releasing at a specific time, i.e., with a time delay or lag time, a pharmaceutical active agent or active agent mixture, e.g., substantially independently of the concentration and type of ions present in the gastro-intestinal environment, e.g., hydrogen ions and hydroxyl ions, i.e., independently of pH, phosphate ions, and also independently of enzymes, present into the surrounding body fluid.

According to the present invention, rivastigmine may be used in the form of the free base or a pharmaceutically acceptable salt thereof. Preferably, the free base is used.

The exact amounts of active agent doses and of the TTS to be administered depend on a number of factors, e.g., the condition to be treated, the desired duration of treatment and the rate of release of active agent.

For example, the amount of the active agent required and the release rate thereof may be determined on the basis of known in vitro or in vivo techniques, determining how long a particular active agent concentration in the blood plasma remains at an acceptable level for a therapeutic effect.

For example, for rivastigmine, dosages in the range of 1 mg to 12 mg of active agent per day for a 70 or 75 kilogram mammal, e.g., humans, and in standard animal models, may be used.

The TTS of the invention allows, e.g., the manufacture of once a day pharmaceutical forms for patients who have to take more than one dose of an active agent per day, e.g., at specific times, so that their treatment is simplified. With such compositions tolerability of rivastigmine may be improved, and this may allow a higher starting dose and a reduced number of dose titration steps.

A increased tolerability of rivastigmine provided by the compositions may be observed in standard animal tests and in clinical trials.

The following numbered paragraphs are of interest to or illustrative of the invention.
1. A Transdermal Therapeutic System (TTS) comprising
   a) a backing layer,
   b) a reservoir layer comprising one or more pharmaceutically active ingredients and one or more polymers,
   c) an adhesive layer comprising a silicone polymer and a tackifier.
2. TTS according to paragraph 1 wherein the backing layer is active ingredient-impermeable
3. TTS according to paragraph 1 or 2 comprising an additional detachable protective layer.
4. A TTS comprising
   a) a backing layer,
   b) a reservoir layer containing at least one pharmaceutically active ingredient in the form of a polymer matrix and
   c) an adhesive layer that has an adhesive force between 10 N/TTS and 100 N/TTS.
5. TTS according to any of paragraphs 1 to 3, **characterized in that** the active ingredient has a saturation solubility of less than 15 %-wt., preferably less than 10%-wt., and most preferred of 2 to 8%-wt., in the silicone adhesive.
6. TTS according to any of paragraphs 1 to 4, **characterized in that** the silicone adhesive layer does reduce active ingredient permeation from the reservoir layer through the skin by no more than 40%.
7. TTS according to any of paragraphs 1 to 5, **characterized in that** the silicone adhesive layer has a weight per unit area in the range of 5 to 60 g/m2.
8. TTS according to any of paragraphs 1 to 6, **characterized in that** the active ingredient is selected from the group consisting of α-adrenoreceptor agonists, β-adrenoreceptor agonists, α-adrenoreceptor blockers, anesthetic analgetics, non-anesthetic analgetics, androgens, anesthetics, antiallergics, antiandrogens, antianginals, antiarrhythmics, penicillins, antidiabetics, antihistaminics, antimigraine agents, hydrated ergot alkaloids, Ca++ antagonists, serotonin antagonists, platelet aggregation inhibitors, antidepressants, broncholytics, estrogens, gestagens, vasodilators, hormones, anti-dementia agent.
9. TTS according to any of paragraphs 1 to 6, **characterized in that** the active ingredient is selected from the group consisting of tacrine, rivastigmine, donepezil, galantamine, physostigmine, huperzine A and pharmacologically acceptable salts thereof.
10. TTS according to any of paragraphs 1 to 6, **characterized in that** the active ingredient is selected from the group consisting of rivastigmine and rivastigmine hydrogentartrate.
11. TTS according to one of the preceding paragraphs, **characterized in that** the reservoir layer comprises a polymer chosen from the group consisting of polydimethylsiloxanes, acrylates, methacrylates, polyisobutylenes, polybutenes and styrene-isoprene-styrene block copolymers or mixtures thereof, respectively combined with resins.
12. TTS according to one of the preceding paragraphs, **characterized in that** the tackifier is selected from the group consisting of Silicone oils, glycerine esters of hydrogenated resin acids, hydroabietyl alcohol, resin esters, Hydrogenated Methyl Ester of Wood Rosin, Ester of Partially Hydrogenated Wood Rosin · Esters of Rosin, etc. and combinations of those.
13. TTS according to paragraph 11, **characterized in that** the additive is chosen from the group consisting of silicone oils and resins.
14. TTS according to one of the preceding paragraphs, **characterized in that** the active ingredient is also contained in the silicone adhesive layer.
15. A TTS comprising as active ingredient rivastigmine in free base or pharmaceutically acceptable salt form and providing a maximum plasma concentration of about 1 to 30 ng/ml from a mean of about 2 to 16 hours after application.
16. A TTS comprising as active ingredient rivastigmine in free base or pharmaceutically acceptable salt form and providing a maximum plasma concentration of about 2.5 to 20 ng/ml from a mean of about 4 to 12 hours after application.
17. A TTS comprising as active ingredient rivastigmine in free base or pharmaceutically acceptable salt form and having an AUC₂₄ₕ of about 25 to 450 ng*h/mL after repeated once daily administration.
18. A TTS comprising as active ingredient rivastigmine in free base or pharmaceutically acceptable salt form and an having an AUC₂₄ₕ of about 45 to 340 ng*h/mL after repeated once daily administration.
19. A TTS according to any of paragraphs 1 to 14 comprising as active ingredient rivastigmine and Memantine.
20. A process for manufacturing a TTS according to any of the preceding paragraphs comprising the steps of
   a) manufacturing of the active ingredient in adhesive solution
   b) coating of the active ingredient in adhesive solution
   c) drying of the active ingredient in adhesive solution
   d) manufacturing of the silicone adhesive solution
   e) coating of the silicone adhesive solution
   f) laminating of the silicone adhesive layer to the drug in adhesive layer
   g) Punching and Pouching.
21. A method for the prevention, treatment or delay of progression of Alzheimer's disease in a subject in need of such treatment, which comprises administering to said subject a therapeutically effective amount of a TTS according to any of paragraphs 1 to 17.
22. A method for the prevention, treatment or delay of progression of dementia associated with Parkinson's disease in a subject in need of such treatment, which comprises administering to said subject a therapeutically effective amount of a TTS according to any of paragraphs 1 to 17.
23. A method for the prevention, treatment or delay of progression of symptoms of traumatic brain injury in a subject in need of such treatment, which comprises administering to said subject a therapeutically effective amount of a TTS according to any of paragraphs 1 to 17.
24. A method for the prevention, treatment or delay of progression of Down's syndrome in a subject in need of such treatment, which comprises administering to said subject a therapeutically effective amount of a TTS according to any of paragraphs 1 to 17.
25. A method for the prevention, treatment or delay of progression of post operative delirium in a subject in need of such treatment, which comprises administering to said subject a therapeutically effective amount of a TTS according to any of paragraphs 1 to 17.
26. Use of a TTS according to any of paragraphs 1 to 17 for prevention, treatment or delay of progression of Alzheimer's disease, dementia associated with Parkinson's disease, symptoms of traumatic brain injury.

The following non-limiting examples illustrate the invention:

### EXAMPLE

### I. TTS Production

The following exemplary tests were conducted using the cholinesterase inhibitor rivastigmine present in form of its free base. For the tests the following two TTSs were produced:
TTS #1: Substrate portions with a weight per unit area of 60 g/m2 having the following composition were produced:

| | |
|---|---|
| rivastigmine (free base) | 30.0 wt-% |
| Durotak® 387-2353 (polyacrylate adhesive) | 49.9 wt-% |
| Plastoid® B (acrylate copolymer) | 20.0 wt-% |
| Vitamine E | 0.1 wt-% |

TTS #2: Substrate portions were produced in the form of a bilayer, one layer of said bilayer corresponding to TTS #1. Said layer is provided with a silicone adhesive layer having a weight per unit area of 30g/m2 according to the following composition:

| | |
|---|---|
| Bio-PSA® Q7-4302 (silicone adhesive) | 98.9 wt-% |
| Silicone oil | 1.0 wt-% |
| Vitamine E | 0.1 wt-% |

The saturation solubility of rivastigmine in form of its free base in the silicone adhesive is about 5%-wt.

### 11. Determination of adhesive force

The adhesive force of both TTSs was determined by methods known to persons skilled in the art taking into consideration the following details:
- Size of substrate portions: 10cm2
- Test plate: steel
- Peeling angle: 90°
- Peeling speed: 300mm/min

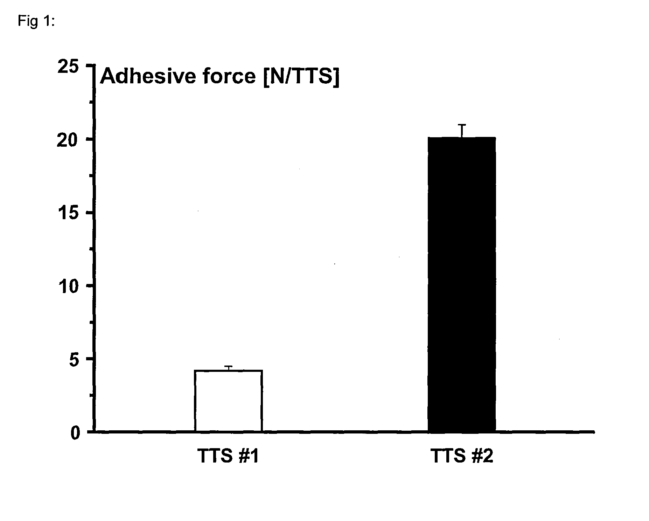

For both TTSs the adhesive forces shown in Figure 1 were obtained. The chart of Figure 1 clearly shows that coating the acrylate adhesive matrix with a silicone adhesive layer significantly increases its adhesive force.

rivastigmine in the form of its free base is liquid at room temperature. It was therefore necessary to add a "thickening polymer" (Plastoid® B) when incorporating 30%-wt. of active ingredient. A substrate with low adhesive force is thus obtained. When using an additional silicone adhesive layer the adhesive force is about five times that of a comparable TTS without additional silicone adhesive layer.

### III. Permeation properties

In order to determine whether the application of an additional silicone adhesive layer affects active ingredient release the permeation of rivastigmine through full-thickness human skin and EVA membranes was tested for both TTSs. For said permeation tests the following conditions applied:

The full-thickness human skin and the EVA membrane were respectively introduced into a modified Franz diffusion cell. The diffusion surface area was 1.51 cm2. Phosphate buffer (pH 5.5) with 0.1% sodium azide was used as acceptor medium. The acceptor medium had a volume of 9ml. The test temperature was adjusted to 32°C by means of a water bath, thus corresponding to the surface temperature of in vivo human skin.

The entire acceptor medium was replaced with fresh acceptor solution after 8, 24, 32, 48, 56 and 72 hours in order to assure perfect sink conditions over the entire test period.

The content of rivastigmine in the acceptor medium was determined by HPLC.

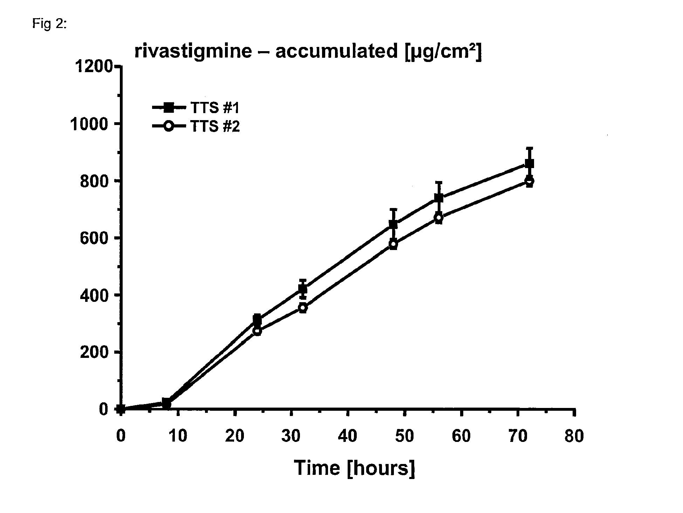

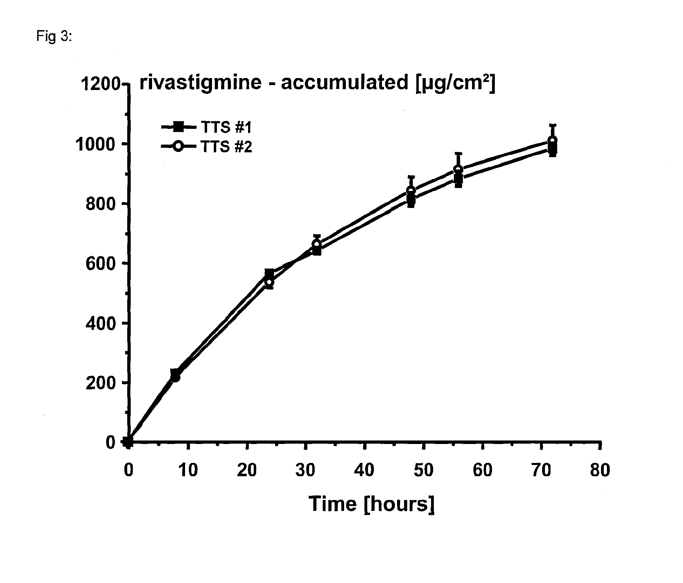

The results of the permeation tests are graphically shown in Figures 2 and 3.

Said results illustrate that practically no differences with regard to permeation rates of rivastigmine present in the form of its free base through human skin were observed between the two TTSs (Figure 2). The slight differences are likely to be due to the use of a biological material like skin and could be explained by local skin variations like for example microlesions or hair follicles.

In order to eliminate variations caused by the use of biological material the permeation tests were repeated using an artificial membrane (EVA membrane). The results shown in Figure 3 confirm the findings obtained with full-thickness human skin, namely that both TTSs do not differ with regard to their permeation properties.

Surprisingly, the application of the additional silicone adhesive layer has no influence on active ingredient permeation through the skin.

According to the present invention TTSs having significantly higher adhesive force while retaining their original size can therefore be produced.

### IV. Pharmacokinetic properties

An open-label, parallel-group, four-period, ascending dose-proportionality study evaluating TTS#2 5 cm², 10 cm², 15 cm², and 20 cm² and 1.5 mg, 3 mg, 4.5 mg, and 6 mg BID Exelon^{®} capsules at steady state in patients with mild-to-moderate Alzheimer's disease was conducted

Patients diagnosed with mild to moderate Alzheimer's Disease were randomized to either TTS#2 or capsule treatment. The criteria for inclusion were: male or female (non-child-bearing potential) patients, 50-85 years of age, who fulfill the (DSM-IV) criteria for dementia of the Alzheimer's type. Patients should have been diagnosed with probable AD according to NINCDS - ADRDA criteria, with a MMSE score of 10-26 (both inclusive), and no other medical conditions that could impact study results.

Based on previous experience in clinical trials, 14-day titration steps were implemented for this study.

At the time of this analysis, the following number of patients completed each of the four periods, and were included in the pharmacokinetic evaluation:

| Capsule | TTS#2 |
|---|---|
| 19 patients in the 1.5 mg bid dose | 18 patients in the 5 cm² dose |
| 18 patients in the 3.0 bid dose | 18 patients in the 10 cm² dose |
| 13 patients in the 4.5 mg bid dose | 16 patients in the 15 cm² dose |
| 12 patients in the 6.0 mg bid dose | 11 patients in the 20 cm² dose |

The pharmacokinetics of rivastigmine were investigated after both treatments on the last day of each titration period, except on highest doses when it is investigated on third day of titration (in order not to miss plasma samplings in case of early drop-outs due to poorer tolerability). Plasma samples were analyzed for rivastigmine using LC-MS/MS with a lower limit of quantification (LLOQ) of 0.2 ng/mL. Standard noncompartmental pharmacokinetic parameters were derived from the individual plasma concentration-time profiles using WinNonlin Pro.

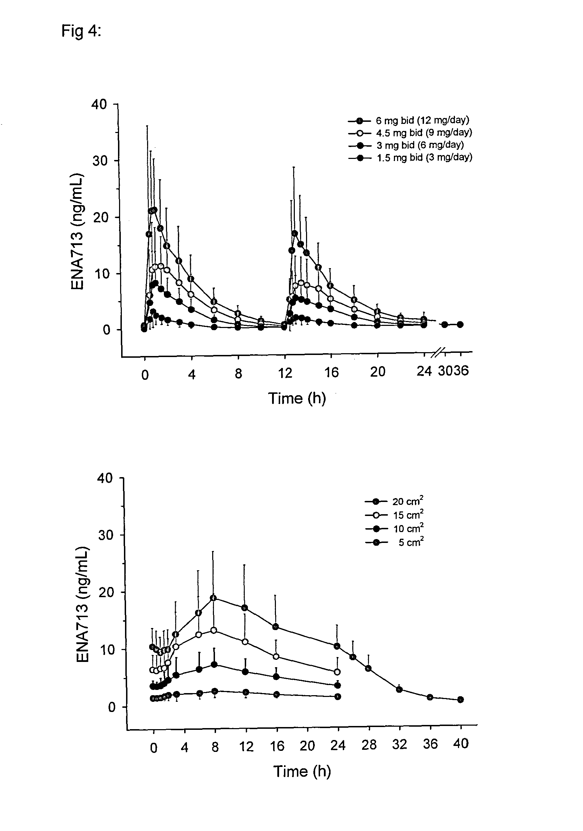

The pharmacokinetic parameters of rivastigmine are summarized in Table 1 (capsule treatment) and Table 2 (TTS#2 treatment). The mean (± SD) plasma concentration-time profiles are displayed in Figure 4.

During the application of TTS#2, a rivastigmine plateau concentration was achieved at a median tₘₐₓ of 8.0 h for all TTS sizes. Exposure also increased over-proportionally with increasing doses as displayed in Table 3, but to a lesser extent than with the capsule, in particular for AUC₂₄ₕ.

The inter-subject variability as assessed by the coefficients of variation (CVs) for the exposure parameters of rivastigmine (Cₘₐₓ and AUC₂₄ₕ) was generally lower after the patch (CVs of 33-48%) as compared to the oral administration (CVs of 39-68%).

### V. Pharmacologic properties

TTS#2 shows improved pharmacological properties when compared with a capsule formulation as shown in standard animal test and in clinical trials..

**Table 1 Descriptive statistics of pharmacokinetic parameters of rivastigmine following capsule administration**

| **ENA713** | Morning dose | | | | | Evening dose | | | | | Daily dose |
|---|---|---|---|---|---|---|---|---|---|---|---|
| | Cₘₐₓ (ng/mL) | tₘₐₓ (h) | AUC₁₂ₕ (ng·h/mL) | AUCₗₐₛₜ (ng·h/mL) | t_{½} (h) | Cₘₐₓ (ng/mL) | tₘₐₓ (h) | AUC₁₂ₕ (ng·h/mL) | AUCₗₐₛₜ (ng·h/mL) | t_{½} (h) | AUC₂₄ₕ (ng·h/mL) |
| **1.5 mg bid (3 mg per day)** | | | | | | | | | | | |
| Mean ± SD | 3.71 ± 2.54 | | 7.01 ± 4.28 | 6.68 ± 4.27 | 1.27 ± 0.25 | 2.3 ± 1.47 | | 5.27 ± 3.31 | 4.94 ± 3.27 | 1.37 ± 0.40 | 12.3 ± 7.41 |
| CV% | 68 | | 61 | 64 | 20 | 62 | | 63 | 66 | 29 | 60 |
| Median | 2.76 | 1.0 | 5.44 | 5.11 | 1.34 | 1.61 | 1.0 | 4.67 | 4.47 | 1.43 | 9.61 |
| Min | 1.17 | 0.5 | 1.99 | 1.84 | 0.75 | 0.901 | 0.5 | 1.64 | 1.44 | 0.55 | 3.63 |
| Max | 10.8 | 3.0 | 18.7 | 18.4 | 1.69 | 5.86 | 4.0 | 13.1 | 12.8 | 2.00 | 31.8 |
| N | 19 | 19 | 19 | 19 | 18 | 19 | 19 | 19 | 19 | 18 | 19 |
| **3 mg bid (6 mg per day)** | | | | | | | | | | | |
| Mean ± SD | 9.82 ± 4.99 | | 29.3 ± 16.4 | 29.0 ± 16.5 | 1.55 ± 0.27 | 7.57 ± 3.90 | | 23.4 ± 13.1 | 23.2 ± 13.1 | 1.74 ± 0.34 | 52.7 ± 29.2 |
| CV% | 51 | | 56 | 57 | 17 | 52 | | 56 | 57 | 20 | 55 |
| Median | 10.5 | 1.0 | 28.1 | 27.7 | 1.62 | 6.59 | 1.0 | 22.1 | 21.7 | 1.72 | 51.8 |
| Min | 2.68 | 0.5 | 8.22 | 8.01 | 0.99 | 2.48 | 0.5 | 6.69 | 6.32 | 1.18 | 17.3 |
| Max | 21.3 | 3.0 | 65.0 | 65.0 | 1.93 | 16.5 | 4.0 | 50.3 | 50.3 | 2.31 | 114 |
| N | 18 | 18 | 18 | 18 | 17 | 18 | 18 | 18 | 18 | 18 | 18 |
| **4.5 mg bid (9 mg per day)** | | | | | | | | | | | |
| Mean ± SD | 15.7 ± 6.68 | | 50.6 ± 25.0 | 50.4 ± 25.1 | 1.73 ± 0.26 | 10.6 ±.12 2 | | 39.8 ± 20.4 | 39.7 ± 20.5 | 2.05 ± 0.46 | 90.4 ± 45.1 |
| CV% | 43 | | 49 | 50 | 15 | 39 | | 51 | 52 | 22 | 50 |
| Median | 15.6 | 1.0 | 46.5 | 46.5 | 1.68 | 10.9 | 1.5 | 38.2 | 38.2 | 2.02 | 84.7 |
| Min | 5.44 | 0.5 | 24.2 | 23.8 | 1.30 | 4.55 | 0.75 | 15.0 | 14.7 | 1.52 | 41.2 |
| Max | 25.5 | 2.0 | 119 | 119 | 2.27 | 19.8 | 6.0 | 93.6 | 93.6 | 3.14 | 213 |
| N | 13 | 13 | 13 | 13 | 13 | 13 | 13 | 13 | 13 | 13 | 13 |
| **6 mg bid (12 mg per day)** | | | | | | | | | | | |
| Mean ± SD | 30.2 ± 14.8 | | 82.1 ± 31.1 | 82.1 ± 35.2 | 1.75 ± 0.24 | 19.3 ± 9.29 | | 68.3 ± 28.2 | 70.0 ± 28.6 | 1.93 ± 0.27 | 150 ± 58.8 |
| CV% | 49 | | 38 | 38 | 14 | 48 | | 41 | 41 | 14 | 39 |
| Median | 26.7 | 0.88 | 72.1 | 72.1 | 1.70 | 18.8 | 1.0 | 60.5 | 62.6 | 1.96 | 129 |
| Min | 12.5 | 0.50 | 35.7 | 35.7 | 1.43 | 8.65 | 0.75 | 30.7 | 30.7 | 1.49 | 66.4 |
| Max | 66.0 | 2.0 | 131 | 131 | 2.24 | 36.9 | 3.0 | 112 | 114 | 2.43 | 242 |
| N | 12 | 12 | 12 | 12 | 12 | 12 | 12 | 12 | 12 | 11 | 12 |

**Table 2 Descriptive statistics of pharmacokinetic parameters of rivastigmine following TTS#2 application**

| **ENA713** | Cₘₐₓ (ng/mL) | tₘₐₓ (h) | AUC₂₄ₕ (ng·h/mL) | AUCₗₐₛₜ (ng·h/mL) | t_{½} (h) |
|---|---|---|---|---|---|
| **5 cm² (9 mg loaded dose)** | | | | | |
| Mean±SD | 2.66 ± 1.15 | | 45.6±16.6 | 45.6±16.6 | na |
| CV% | 43 | | 36 | 36 | na |
| Median | 2.66 | 8.0 | 48.3 | 48.3 | na |
| Min | 1.19 | 0.5 | 19.7 | 19.7 | na |
| Max | 4.63 | 12.0 | 74.9 | 74.9 | na |
| N | 18 | 18 | 18 | 18 | na |
| **10 cm² (18 mg loaded dose)** | | | | | |
| Mean ± SD | 7.57 ± 2.74 | | 123 ± 41.0 | 123 ± 41.0 | na |
| CV% | 36 | | 33 | 33 | na |
| Median | 7.75 | 8.0 | 121 | 121 | na |
| Min | 2.76 | 3.0 | 58.5 | 58.5 | na |
| Max | 12.0 | 16.0 | 199 | 199 | na |
| N | 18 | 18 | 18 | 18 | na |
| **15 cm² (27 mg loaded dose)** | | | | | |
| Mean±SD | 13.8 ± 6.58 | | 226 ± 85.5 | 226 ± 85.5 | na |
| CV% | 48 | | 38 | 38 | na |
| Median | 15.6 | 8.0 | 243 | 243 | na |
| Min | 4.32 | 3.0 | 93.6 | 93.6 | na |
| Max | 25.7 | 16.0 | 346 | 346 | na |
| N | 16 | 16 | 16 | 16 | na |
| **20 cm² (36 mg loaded dose)** | | | | | |
| Mean ± SD | 19.0 ± 8.04 | | 339 ± 138 | 397 ± 154 | 3.40 ± 0.67 |
| CV% | 42 | | 41 | 39 | 20 |
| Median | 17.1 | 8.0 | 323 | 368 | 3.24 |
| Min | 7.55 | 0.0 | 140 | 180 | 2.60 |
| Max | 33.7 | 12.0 | 529 | 598 | 4.62 |
| N | 11 | 11 | 11 | 11 | 11 |

| | | | | | |
|---|---|---|---|---|---|
| na = not available | | | | | |

**Table 3. Extent of rivastigmine exposure increase with increasing dose**

| **rivastigmine** | **Capsule** | | **TTS#2** | |
|---|---|---|---|---|
| **Dose** | **Cₘₐₓ** | **AUC₂₄ₕ** | **Cₘₐₓ** | **AUC₂₄ₕ** |
| **x 2** | **x 2.6** | **x 4.3** | **x 2.8** | **x 2.7** |
| **x 3** | **x 3.8** | **x 7.3** | **x 5.2** | **x 5.0** |
| **x 4** | **x 7.3** | **x 12.2** | **x 7.1** | **x 7.4** |

## Claims

1. A Transdermal Therapeutic System (TTS) comprising
a) a backing layer,
b) a reservoir layer comprising one or more pharmaceutically active ingredients and one or more polymers,
c) an adhesive layer comprising a silicone polymer and a tackifier.

2. TTS according to claim 1 wherein the backing layer is active ingredient-impermeable

3. TTS according to claim 1 or 2 comprising an additional detachable protective layer.

4. A TTS comprising
a) a backing layer,
b) a reservoir layer containing at least one pharmaceutically active ingredient in the form of a polymer matrix and
c) an adhesive layer that has an adhesive force between 10 N/TTS and 100 N/TTS.

5. TTS according to any of claims 1 to 4, **characterized in that** the silicone adhesive layer does reduce active ingredient permeation from the reservoir layer through the skin by no more than 40%.

6. TTS according to any of claims 1 to 4, **characterized in that** the silicone adhesive layer has a weight per unit area in the range of 5 to 60 g/m2.

7. TTS according to any of claims 1 to 5, **characterized in that** the active ingredient is selected from the group consisting of rivastigmine and rivastigmine hydrogentartrate.

8. TTS according to one of the preceding claims, **characterized in that** the reservoir layer comprises a polymer chosen from the group consisting of polydimethylsiloxanes, acrylates, methacrylates, polyisobutylenes, polybutenes and styrene-isoprene-styrene block copolymers or mixtures thereof, respectively combined with resins.

9. TTS according to one of the preceding claims, **characterized in that** the tackifier is selected from the group consisting of Silicone oils, glycerine esters of hydrogenated resin acids, hydroabietyl alcohol, resin esters, Hydrogenated Methyl Ester of Wood Rosin, Ester of Partially Hydrogenated Wood Rosin Esters of Rosin, etc. and combinations of those.

10. TTS according to one of the preceding claims, **characterized in that** the active ingredient is also contained in the silicone adhesive layer.

11. Use of a TTS according to any of claims 1 to 10 for prevention, treatment or delay of progression of Alzheimer's disease, dementia associated with Parkinson's disease, symptoms of traumatic brain injury.

12. A TTS according to claim 1 wherein the TTS has an adhesive force > 5N/10cm².

13. A TTS according to claim 12 wherein the TTS has an adhesive force > 10N/10cm².

14. A TTS according to claim 1 wherein the TTS has an adhesive force < 100N/10cm².

15. A TTS according to claim 14 wherein the TTS has an adhesive force < 50N/10cm².

16. A TTS according to claim 1 wherein the weight per unit area of the silicone adhesive layer is in the range of 5 to 60 g/m², preferably in the range of 10 to 30 g/m².

17. A TTS according to claim 1 wherein the TTS has a size range of 2 to 50 cm², particularly preferred 5 to 20 cm².

18. A TTS according to any one of claims 1 or 4, having as active ingredient rivastigmine in free base or pharmaceutically acceptable salt form having a mean maximum plasma concentration of about 1 to 30 ng/ml from a mean of about 2 to 16 hours after application and an AUC 24h of about 25 to 450 ng*h/mL after repeated "QD" (i.e. once daily) administration.
